# EUROPEAN PATENT SPECIFICATION

(11) **EP 4 041 413 B1**
(45) Date of publication and mention of the grant of the patent: **25.09.2024**
(21) Application number: 21725496.0
(22) Date of filing: 10.06.2021
(51) Int. Cl.: A62B 18/02, A62B 23/02

(54) **ANTI-VIRUS AIR PURIFYING DEVICE**
ANTI-VIRUS-LUFTREINIGUNGSVORRICHTUNG
DISPOSITIF DE PURIFICATION D'AIR ANTI-VIRUS

(30) Priority: 22.06.2020 US 202062705325 P
(43) Date of publication of application: 17.08.2022
(73) Proprietor: Stanley Black & Decker, Inc., New Britain, CT 06053 (US)
(72) Inventor: VANJANI, Govind, W., Danbury, CT 06811 (US); MAYBURY, Mark, T., Chelmsford, MA 01824 (US); GORTI, Bhanuprasad, V., Perry Hall, MD 21128 (US); WELSH, Robert, P., Phoenix, MD 21131 (US); VAN IDERSTINE, Sky, R., Davidsonville, MD 21035 (US); AYALA, Adan, Idlewylde, MD 21239 (US); WINK, Elizabeth, G., Baltimore, MD 21230 (US)
(74) Representative: SBD IPAdmin
(86) International application number: PCT/EP2021/062534
(87) International publication number: WO 2021/259552

(56) References cited:
- CN-A- 101 623 134
- CN-A- 111 227 358
- CN-A- 111 227 359
- GB-A- 2 600 136

## Description

### FIELD OF THE INVENTION

The present invention relates to an anti-virus air purifying device and, more particularly, to an anti-virus mask that automatically counters viruses (e.g., SARS, MERS, Cov-SARS-1, Cov-SARS-2) by employing a multiplicity of filtering and neutralizing components, included viruses, bacteria and fungi.

### BACKGROUND OF THE INVENTION

A conventional anti-virus mask comprises a mask body and two loop-shaped straps mounted on two opposite ends of the mask body. One of the many functions of the mask is to prevent the distribution and spread of active virus and virus particles into the surrounding air. Because an infected person breathing, coughing or sneezing can spray virus particles into the surrounding air, the spread of such particles can be minimized by an infected person wearing a mask.

When in use, the straps are put on a user's ears, and the mask body is placed over the user's mouth and nose, the mask will stop virus particles from travelling far from the user and infecting another person, as well as preventing the virus particles from touching and entering the user's mouth and nose. It is highly desirable to prevent virus particles from entering the user's mouth and nose as the virus particles cannot survive without a host and can only reproduce by attaching themselves to the user's cells.

Typical masks however may not be able to prevent a virus particle from entering the user's mouth and/or nose, resulting in risk of damage or infection to the user. For example, the particle size of SARS-CoV-2 virus (hereinafter "COVID-19") ranges from 0.06 to 0.14 microns, i.e., about 0.1µm on average. In clinical tests of the ability of different mask types to filter 0.007µm particles (ten times smaller than COVID-19), it was found that 3.4% of the particles passed through an N95 mask, 20% of particles passed through a surgical mask and a 72% of particles passed through a cotton handkerchief (Langrish et al. 2009).

Examples of known masks used to neutralize viruses are found, for example, in CN 111227358 and CN 111227359. Whilst these documents disclose masks with thermal layers heating in order to neutralize viruses, their temperatures are maintained at a relatively low level so as not to burn the wearer of the mask.

Accordingly, there is a literal and figurative gap in protecting users from dangerous bacteria and viruses. It is an object of the invention to provide a mask that minimizes the number of bacteria, virus and fungi particles that travel through the ambient air and reach and enter the user's mouth and/or nose. The invention is defined by the appended claims.

### BRIEF DESCRIPTION OF THE DRAWINGS

FIG. 1 is a side view of a first embodiment of the mask.
FIG. 2 is a partial cross-sectional along line A-A of FIG. 1.
FIG. 3 is a side view of a second embodiment of the mask.
FIG. 4 is a partial cross-sectional along line B-B of FIG. 3.
FIG. 5 is a circuit diagram of several elements of the mask of FIG. 3.
FIG. 6 is a perspective view of a powered air-purifying respirator (PAPR), not forming part of the present invention.
FIG. 7 is a side view of an air purifier system, not forming part of the present invention.
FIG. 8 is a partial side view of a second air purifier system, not forming part of the present invention.
FIG. 9 is a partial side view of a third air purifier system, not forming part of the present invention.

### DETAILED DESCRIPTION

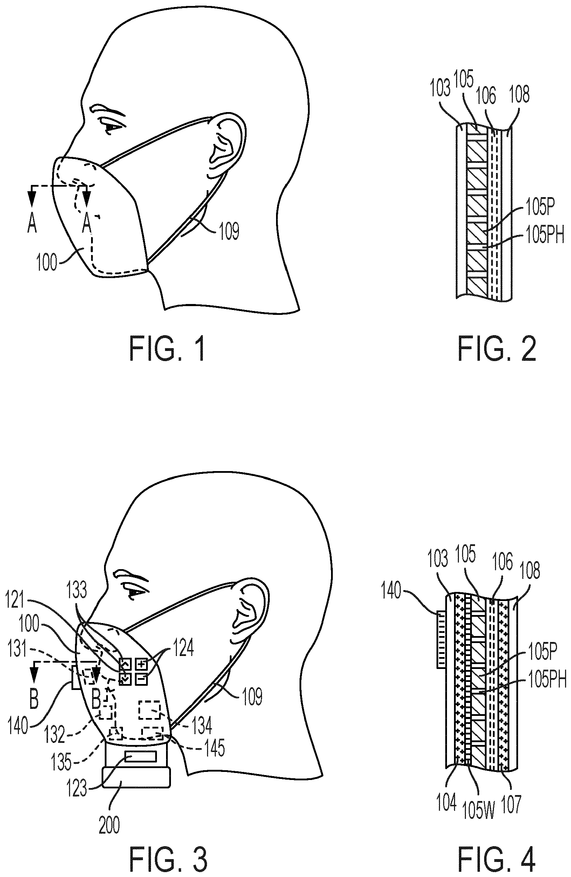

Referring to FIGS. 1-2, a first embodiment of a mask 100 may include an outer layer 103, a biocidal layer 105, a filtering layer 106, and an inner layer 108 contacting the user's skin. Mask 100 may also have straps 109 for strapping mask 100 unto the user's head. Preferably straps 109 are loop-shaped to facilitate on a user's ears or around the user's head. Persons skilled in the art are referred to US Publication No. US2011/0296584.

Preferably the outer layer 103 is made of a mixed woven fiber cloth to isolate dust and bacteria having a larger size. Preferably, the outer layer 103 is provided with an additive, such as a water-repellent agent, a photocatalyst, a nano-silver antibacterial agent, a copper fiber antibacterial material, and the like. Thus, when the outer layer 103 is provided with a water-repellent agent, the outer layer 103 can enhance the waterproof effect of the mask, and when the outer layer 103 is provided with a photocatalyst or a nano-silver antibacterial agent, or antibacterial copper fiber, the outer layer 103 can provide a sterilizing effect to neutralize and/or destroy bacteria and viruses.

Outer layer 103 may also be made of a heat-moldable fabric, such as a heat-moldable fabric selected from the group consisting of polypropylene, polyester and non-woven cellulose acetate fabric. Such heat-moldable fabrics permit shaping of facial masks with heat or ultrasonic welding according to the present invention. In one embodiment, the heat-moldable fabric comprises polypropylene webbing which traps airborne particles, but is relatively water repellent so that virus-laden droplets are normally not disrupted even if the virus-laden droplets are trapped within the webbing.

Outer layer 103 made alternatively be made of a material, such as e-PTFE (expanded polytetra-fluoro-ethylene), TPE (thermoplastic elastomer), TPEE (thermoplastic polyester/polyether elastomer), COC (cyclic olefin copolymer), FRP (fiber reinforced plastic) and the like. Outer layer 103 would be provided with pores having a size smaller than that of a dust, water molecule, bacterium and virus to isolate the dust, water molecule, bacterium and virus efficiently so as to prevent the dust, water molecule, bacterium and virus from passing through outer layer 103.

Mask 100 may also have a biocidal layer 105. Biocidal layer 105 may be a copper mesh layer or a copper plate 105P with holes 105PH. Preferably the holes are between 0.1µm and 0.3µm. The copper plate 105P may be a flexible plate to ensure the mask 100 better conforms to the user's face shape. Persons skilled in the art shall recognize that the copper in biocidal layer 105 may not be a pure form of copper, but instead may be phosphate glass fibers impregnated with copper oxide, glass coated with thin films of copper oxide, metallic and copper alloys, or fabric fibers impregnated with copper compounds. Such biocidal layer 105 would provide antiviral properties to mask 100.

Mask 100 may also have a filtering layer 106 made of a common fiber non-woven cloth to provide a ventilating effect and to utilize static electricity to catch bacteria, dust and viruses moving through the mask 100. Preferably the cloth layer is made of non-woven cloth having a filtration rating between N90 and N100.

An inner layer 108 may be provided adjacent to filtering layer 106 for contacting the user's skin. Preferably inner layer 108 is made of cloth. Such layer can provide a warming effect to the user when the ambient environment has a lower temperature.

FIGS. 3-5 illustrate a second embodiment of a mask 100, where like numerals refer to like parts. All the teachings from the previous embodiment are hereby incorporated. Mask 100 may include an outer layer 103, a first thermal layer 104, a biocidal layer 105, a filtering layer 106, a second thermal layer 107, an inner layer 108 contacting the user's skin, and straps 109.

As in the first embodiment, mask 100 may have biocidal layer 105, with a copper mesh layer or a copper plate 105P with holes 105PH. Preferably the holes 105PH are between 0.1µm and 0.3µm. A heating wire or thermal pad 105W may thermally contact the copper plate 105P to heat copper plate 105P to a temperature of at least 75 degrees Celsius (and preferably at least 180 degrees Celsius) to neutralize viruses, such as the COVID-19 virus. Such neutralization may be accomplished by destroying the S2/S-protein of the COVID-19 virus (which may be involved in receptor recognition, viral attachment and entry into cell) (https://www.nature.com/articles/s41401-020-0485-4). Alternatively, thermal pad 105W may be used without copper plate 105P. Persons skilled in the art shall recognize that heating/radiation sources other than thermal pad 105W could be used to generate the heat or radiation, such as a power transistor or diode on or with a heat sink, ultraviolet light sources, microwave, induction, etc. Furthermore, it may obviate the need for a separate filtered exhaust.

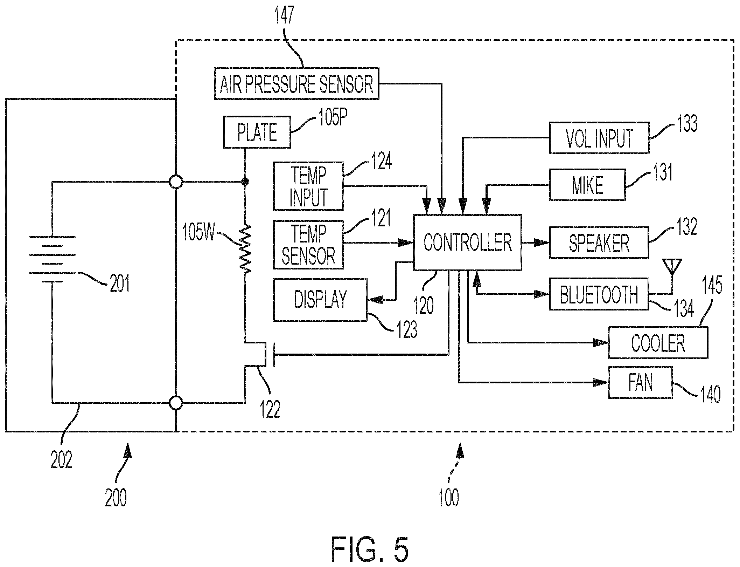

Referring to FIGS. 3 and 5, a battery pack 200 may be attached to mask 100. A user would be able to charge battery pack 200 by connecting it to a USB charger. Such battery pack 200 could be a pouch battery pack.

Alternatively, battery pack 200 may be removably attached to mask 100. Preferably battery pack 200 is a power tool battery pack. Persons skilled in the art shall understand that "power tool battery pack" as used herein shall mean a set of rechargeable battery cells 201 disposed in a housing 202 that for use with a tool that is powered by an electrical motor, such as a drill, circular saw, reciprocating saw, jigsaw, etc. Persons skilled in the art shall recognize that power tool battery pack 200 may be the power tool battery packs disclosed in U.S. Pat. Nos. 7,405,536, 7,618,741, 7,602,146 and/or 8,044,640.

In particular battery pack 200 may be electrically connected to thermal pad 105W. In order to ensure that thermal pad 105W is heated to the desired temperature, mask 100 preferably has a controller 120, which receives a signal representative of the temperature of thermal pad 105W from temperature sensor 121. Controller 120 can then adjust the amount of current received by thermal pad 105W by controlling a transistor 122 using a PWM current control technique. Controller 120 can also adjust the temperature of thermal pad 105W dependent upon the input provided by the user via the temperature input buttons 124. Persons skilled in the art will recognize that it is preferable to ensure that thermal pad 105W is heated to a minimum temperature threshold, and that the user can adjust the temperature of thermal pad 105W higher than such temperature threshold.

Persons skilled in the art shall recognize that it may be advantageous to have an air pressure sensor 147 to sense the air pressure between the mask 100 and the user's face. The air pressure sensor 147 can provide such pressure information to controller 120, which can use this information to increase or decrease the temperature of thermal pad 105W or increase the speed of a thermoelectric cooler 145 (as described below) depending upon the increased or decreased pressure caused by the user's respiration.

Controller 120 preferably controls display 123. Display 123 preferably shows the temperature of thermal pad 105W and/or signals whether mask 100 is ready for use by providing a color signal, such as displaying "red," "yellow," and "green" colors meaning "not ready," "almost ready" and "ready for use," respectively.

Persons skilled in the art shall recognize that battery pack 200 may also be provided with a belt clip so that the user would not have to carry the battery pack 100 on mask 100. Instead a cable (not shown) extending between battery pack 200 and mask 100 would be provided to electrically connect battery pack 200 and mask 100.

Persons skilled in the art shall recognize that plate 105P may be connected to the positive output of battery cells 201 to electrically charge plate 105P with a positive charge, as shown in FIG. 5. Because the COVID-19 particles are negatively charged, they will be attracted to the positively-charged plate 105P, minimizing the number of particles that move beyond plate 105P into the user's nose and/or mouth. Alternatively, plate 105P may be connected to the negative output of battery cells 201 to electrically charge plate 105P with a negative charge. Because the COVID-19 particles are negatively charged, they will be repelled by the negatively-charged plate 105P, moving such particles away from the user.

Plate 105P may be electrically charged to a high voltage (at least 3000-6000 volts and preferably at least 30 kilovolts) with a high voltage power supply, such as high voltage power supply HVP or power supply control DC described below.

Referring to FIG. 4, mask 100 may also have thermal layers 104, 107 disposed adjacent to outer layer 103 and inner layer 108, respectively. Preferably, the thermal layers 104, 107 will be made of a thermally insulating material, such as microfiber. Persons skilled in the art will recognize that it is desirable to have thermal layer 104 between the outer layer 103 and biocidal layer 105 to enable the user to comfortably touch outer layer 103. Similarly, it is desirable to have thermal layer 107 between the inner layer 108 and biocidal layer 105 to enable the user's face to comfortably touch inner layer 108.

Alternatively, thermal layer 104 may be made of a metal so as to act as cooling heat sink for biocidal layer 105, so that the user does not feel the heat generated by biocidal layer 105.

Persons skilled in the art may recognize that mask 100 may attenuate a user's voice when communicating while wearing the mask 100. Accordingly, it is desirable to provide some means for facilitating communications.

Referring to FIGS. 3 and 5, mask 100 may have a microphone 131. Controller 120 may receive an audio signal from microphone 131 and send it to a speaker 132 disposed on the mask 100. Speaker 132 could be a piezoelectric speaker.

Speaker 132 is preferably amplified so that the user can project his or her voice at a louder volume than his or her usual speaking voice. Preferably the user can control the speaker volume using the volume input controls 133. Persons skilled in the art will recognize speaker 132 is preferably powered by battery pack 200.

Controller 120 can also send the audio signal to a wireless communication module, such as Bluetooth transceiver 134, which can be wirelessly connected to a separate speaker. Persons skilled in the art will recognize Bluetooth transceiver 134 is preferably powered by battery pack 200.

To improve the clarity of the amplified user's voice, it may be advantageous to add sound dampening on inner layer 108. Sound dampener 135 could be made of fabric, soundproof foam, etc. Preferably the material of sound dampener 135 is selected to absorb high and/or low frequencies, e.g., below approximately 300Hz and/or above 5kHz. Persons skilled in the art shall recognize that microphone 131 (and/or an additional microphone (not shown)) may sense ambient sound, which can be used by controller 120 to generate an opposite sound wave to output to speaker 132. Such opposite sound wave would cancel the ambient sound, improving the clarity of the amplified user's voice.

Referring to FIGS. 3-5, mask 100 may also carry a small fan 140 for blowing away floating droplets near the user as well as for cooling air heated by the user's breath or thermal pad 105W. Persons skilled in the art will recognize fan 140 is preferably powered by battery pack 200. The speed of fan 140 may be controlled by controller 120 based on user input.

Mask 100 may also carry a thermoelectric cooler 145 for lowering the temperature inside mask 100. Persons skilled in the art will recognize that thermoelectric cooler 145 is preferably a Peltier device. Thermoelectric cooler 145 is preferably powered by battery pack 200. The temperature of thermoelectric cooler 145 may be controlled by controller 120 based on user input. Persons skilled in the art shall recognize that a Peltier device can also be used to warm the space inside the mask 100, which could be desirable for a user working in a cold environment.

Persons skilled in the art may recognize that, depending upon the desired use or application of mask 100, it may be desirable to add ribs or seals on the inside of mask 100 in order to prevent any air movement between the nose and the mouth within mask 100. Such arrangement can be found in US Publication No. 20170157435.

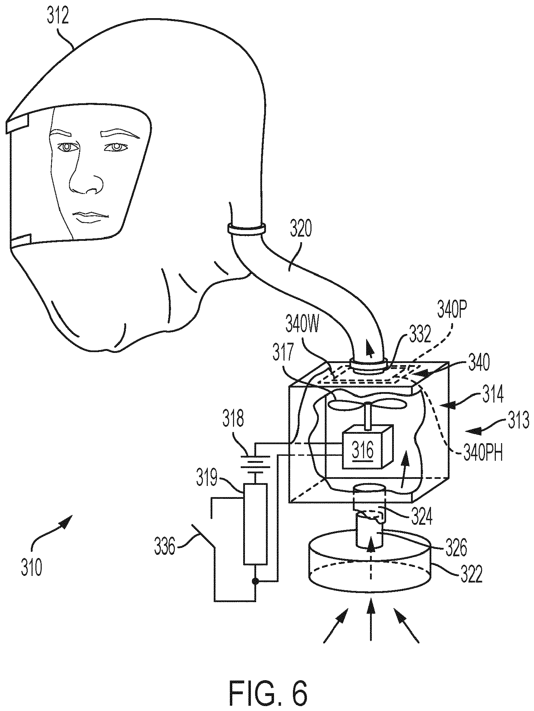

FIG. 6 illustrates a powered air-purifying respirator (PAPR) 310 , not forming part of the present invention, but incorporating some of the teachings of the previous embodiments. The PAPR 310 preferably delivers a volume of purified air at a generally constant flow rate regardless of changes in the configuration of its elements, the operating condition of the system, or the environment in which the apparatus is used. The air flow can be increased when a sensor indicates either increased heat in the chamber or increase in respiration rate during heavier workloads to provide a larger volume of air.

PAPR 310 preferably includes an air delivery system having a filter bank 322 for removing harmful particulate matter or gas from the air in a particular environment. The filter bank 322 is attached to a blower assembly 313 by way of fittings 324 on a connecting conduit 326 from the filter bank to the blower housing 314. A motor 316 drives a turbine 317 that draws air through the filter bank 322 and delivers it by way of a hose 320 to a contained wearer environment, such as a face piece, a head piece or a suit 312 worn by the user. Voltage to the motor 316 is supplied by a battery pack 318 through a controller 319 that regulates power to the blower motor 316 in response to control signal inputs from a microprocessor integrated into the controller 319. The microprocessor monitors a switch 336 to determine whether to apply electrical power to the controller 319 and motor 316. Persons skilled in the art are referred to US Patent Nos. 10,441,828 and 6,666,209.

PAPR 310 may have a biocidal filter 340, with a copper mesh layer or a copper plate 340P with holes 340PH or a copper nanofibre. Preferably the holes 340PH are between 0.1µm and 0.3µm. A heating wire or thermal pad 340W may thermally contact the copper plate 340P to heat copper plate 340P to a temperature of at least 75 degrees Celsius (and preferably at least 180 degrees Celsius) to neutralize viruses. Alternatively, thermal pad 340W may be used without copper plate 340P. Persons skilled in the art shall recognize that heating/radiation sources other than thermal pad 340W could be used to generate the heat or radiation, such as a power transistor or diode on or with a heat sink, ultraviolet light sources, microwave, induction, etc.

Battery pack 318 may be connected to thermal pad 340W. In addition, plate 340P may be connected to the positive output of battery pack 318 to electrically charge plate 340P with a positive charge. Because the COVID-19 particles are negatively charged, they will be attracted to the positively-charged plate 340P, minimizing the number of particles that move beyond plate 340P into hose 320 and ultimately into the user's nose, mouth and/or eyes. Alternatively, plate 340P may be connected to the negative output of battery pack 318 to electrically charge plate 340P with a negative charge. Because the COVID-19 particles are negatively charged, they will be repelled by the negatively-charged plate 340P, moving such particles away from the hose 320.

Plate 340P may be electrically charged to a high voltage (at least 3000-6000 volts and preferably at least 30 kilovolts) with a high voltage power supply, such as high voltage power supply HVP or power supply control DC described below.

Persons skilled in the art shall recognize that the arrangement of PAPR 310 can be installed in a fully or partially sealed room or building or land, air, sea or space vehicle to provide purified air to such a location. As such, thermal pad 340W and/or plate 340P could be connected to battery pack 318 as described above. Alternatively thermal pad 340W and/or plate 340P could be connected to an AC source.

Persons skilled in the art shall recognize that it is desirable to construct mask 100 and/or PAPR 310 using zero defect or six sigma manufacturing processes and techniques.

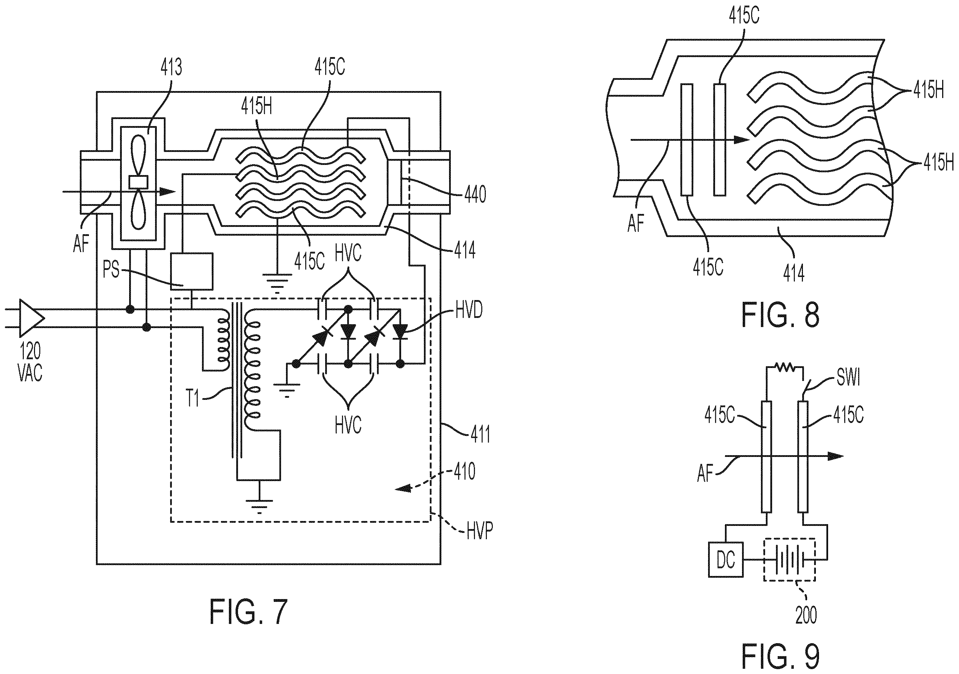

FIG. 7 illustrates a powered air purifier system 410 , not forming part of the present invention, but incorporating some of the teachings of the previous embodiments. The air purifier system 410 preferably delivers a volume of purified air at a generally constant flow rate regardless of changes in the configuration of its elements, the operating condition of the system, or the environment in which the apparatus is used. The air flow can be increased when a sensor indicates either increased heat in the chamber or increase in respiration rate during heavier workloads to provide a larger volume of air.

Air purifier system 410 preferably includes a housing 411 enclosing and/or supporting several components, including a blower assembly 413. Blower assembly 413 preferably includes an electric fan for moving air along air flow AF. Persons skilled in the art shall recognize that the electric fan in blower assembly 413 may be powered by AC, as is well known in the art.

An air processing chamber 414 is preferably disposed downstream of blower assembly 413. Processing chamber 414 preferably has a plurality of copper plates, including heater plates 415H and/or electrostatic plates 415C.

As discussed above, a heating wire or thermal pad may thermally contact the heating plates 415H to heat heating plates 415H to a temperature of at least 75 degrees Celsius (and preferably at least 180 degrees Celsius) to neutralize viruses. Such heating wire or thermal pad may be connected to a power supply PS connected to the AC source. Persons skilled in the art shall recognize that heating/radiation sources other than a thermal pad could be used to generate the heat or radiation, such as a power transistor or diode on or with a heat sink, ultraviolet light sources, microwave, induction, etc.

Heating plates 415H are preferably undulating and parallel to each other, allowing the air to go through heating plates 415H. Heating plates 415H may be disposed at a distance of at least 0.5mm and preferably less than 5mm from each other. The airflow through heating plates 415 is at least 0.25 cfm (cubic feet per minute), and preferably less than 15 cfm (1 cfm = 28.3 liter per minute).

As mentioned above, processing chamber 414 may also have electrostatic plates 415C. Preferably one electrostatic plate 415C will be connected to an output of a high voltage power supply HVP to electrically charge electrostatic plate 415C with a positive charge while another electrostatic plate 415C will be connected to ground. Alternatively, one electrostatic plate 415C will be connected to a positive output of the high voltage power supply HVP to electrically charge electrostatic plate 415C with a positive charge while the other electrostatic plate 415C will be connected to a negative output of the high voltage power supply HVP to electrically charge electrostatic plate 415C with a negative charge. Because the COVID-19 particles are negatively charged, they will be attracted to the positively-charged electrostatic plate 415C, minimizing the number of particles that move beyond electrostatic plate 415C (and thus downstream) and ultimately into the user's nose, mouth and/or eyes.

As shown in FIG. 7, electrostatic plates 415C may sandwich the heating plates 415H therebetween with electrostatic plates 415C and heating plates 415H being within air processing chamber 414. Alternatively electrostatic plates 415C may be disposed upstream (relative to air flow AF) from the heating plates 415H. Electrostatic plates 415C and heating plates 415H may be within air processing chamber 414.

As shown in FIG. 8, electrostatic plates 415C may be disposed perpendicularly to air flow AF. It is preferable to connect the first electrostatic plate 415C along air flow AF to a negative potential (i.e., -V) or ground, while connecting the second electrostatic plate 415C along air flow AF to a positive potential (i.e., +V) or ground (if the first electrostatic plate 415C along air flow AF to the negative potential -V).

Preferably electrostatic plates 415C may be porous so as to allow some air flow AF to continue to move towards heating plates 415H. The porosity of electrostatic plates 415C may be selected to match the air flow allowed by a standard N95 or KN95 masks..

It is desirable to electrically charge electrostatic plate 415C to a high voltage (at least 3000-6000 volts and preferably at least 30 kilovolts).

High voltage power supply HVP can be used to provide such voltage. High voltage power supply HVP preferably includes a transformer T1 that can convert 120VAC to 3000-6000VAC. If necessary, a high voltage multiplier can be disposed to step up the output of transformer T1 to the desired voltage. FIG. 7 shows a typical high voltage multiplier that can be made with the following components: high voltage capacitors HVC (e.g., 470 picofarad, 20 kilovolt ceramic doorknob capacitors) and high voltage diodes HVD (e.g., HV03-12 12 kilovolt peak inverse voltage (PIV) high voltage diodes).

Alternatively, a battery pack 200 (and possibly a power tool battery pack) can be used to provide the charge the electrostatic plates 415C as shown in FIG. 9. A switch SW1 can be used to connect battery pack 200 to the electrostatic plates 415C, If multiple battery packs 200 are used, it may be preferable for switch SW1 to have multiple poles so as to connect multiple battery packs 200 to the electrostatic plates 415C simultaneously.

A power supply control DC can be used to control the voltage delivered to electrostatic plates 415C. Persons skilled in the art shall recognize that power supply control DC may also include multiple high voltage capacitors (not shown) connected in parallel for charging, and then connected in series via a switch (not shown) for discharging, as is well known in the art, thus increasing the effective charging potential of electrostatic plates 415C.

The switch SW1 may also be a double throw switch so that it can be used to reverse the polarity of electrostatic plates 415C. By reversing the polarity of electrostatic plates 415C, the negatively charged COVID-19 particles will be repelled by the now-negative electrostatic plate 415C, moving downstream towards heating plates 415H. This effectively clears electrostatic plates 415C of any build-up.

Persons skilled in the art shall recognize that the high voltages provided by the power supplies shown in FIGS. 7-9 may ionize the ambient air within air processing chamber 414. Such ionization is advantageous as it may reduce particulates, microbes and odors.

Air purifier system 410 may have a biocidal filter 440, with a copper mesh layer or a copper plate with holes or a copper nanofiber. Preferably the holes are between 0.1µm and 0.3µm.

Persons skilled in the art shall recognize that the arrangement of air purifier system 410 can be a stand-alone unit installed in a fully or partially sealed room or within a central HVAC system of a building or land, air, sea, subsurface or space vehicle to provide purified air to and within such a location.

Persons skilled in the art shall recognize that it is desirable to construct air purifier system 410 using zero defect or six sigma manufacturing processes and techniques.

While preferred embodiments of this disclosure have been described above and shown in the accompanying drawings, it should be understood that applicant does not intend to be limited to the particular details described above and illustrated in the accompanying drawings but only to the scope of the appended claims.

## Claims

1. A mask (100, 312) comprising:
an outer layer (103),
a filtering layer (106), a biocidal layer (105),
a thermal element (105W), an inner layer (108) contacting user's skin, and
at least one strap (109) for securing the mask on the user,
the mask **characterised in that** the thermal element (105W) heats air to a temperature of at least 180 degrees Celsuis.

2. The mask of Claim 1, wherein the thermal element is connected to a battery pack.

3. The mask of Claim 2, wherein the battery pack is a power tool battery pack.

4. The mask of Claim 1, wherein the biocidal layer comprises at least one of a copper mesh and a copper plate.

5. The mask of Claim 1, further comprising at least one of an air pressure sensor (147), a display, a fan (140), a thermoelectric cooler (145), a microphone (131) and a speaker (132).

## Patentansprüche

1. Maske (100, 312), umfassend:
eine Außenschicht (103),
eine Filterschicht (106),
eine biozide Schicht (105),
ein Thermoelement (105W), eine Innenschicht (108), die mit einer Benutzerhaut in Berührung ist, und
mindestens einen Riemen (109) zum Sichern der Maske am Benutzer,
wobei die Maske **dadurch gekennzeichnet ist, dass** das Thermoelement (105W) Luft auf eine Temperatur von mindestens 180 Grad Celsius erwärmt.

2. Maske nach Anspruch 1, wobei das Thermoelement mit einem Batteriesatz verbunden ist.

3. Maske nach Anspruch 2, wobei der Batteriesatz ein Batteriesatz für ein Elektrowerkzeug ist.

4. Maske nach Anspruch 1, wobei die biozide Schicht mindestens eines umfasst von einem Kupferdrahtgeflecht und einer Kupferplatte.

5. Maske nach Anspruch 1, weiter umfassend mindestens eines von einem Luftdrucksensor (147), einer Anzeige, einem Ventilator (140), einem thermoelektrischen Kühler (145), einem Mikrofon (131) und einem Lautsprecher (132).

## Revendications

1. Masque (100, 312) comprenant :
une couche externe (103),
une couche filtrante (106),
une couche biocide (105),
un élément thermique (105W), une couche interne (108) en contact avec la peau d'un utilisateur, et
au moins une lanière (109) pour fixer le masque sur l'utilisateur,
le masque étant **caractérisé en ce que** l'élément thermique (105W) chauffe l'air à une température d'au moins 180 degrés Celsius.

2. Masque selon la revendication 1, dans lequel l'élément thermique est connecté à un bloc batterie.

3. Masque selon la revendication 2, dans lequel le bloc-batterie est un bloc-batterie d'outil électrique.

4. Masque selon la revendication 1, dans lequel la couche biocide comprend au moins l'un d'un maillage en cuivre et d'une plaque de cuivre.

5. Masque selon la revendication 1, comprenant en outre au moins l'un parmi un capteur de pression d'air (147), un écran, un ventilateur (140), un refroidisseur thermoélectrique (145), un microphone (131) et un haut-parleur (132).
